# EUROPÄISCHE PATENTSCHRIFT

(11) **EP 0 998 441 B1**
(45) Veröffentlichungstag und Bekanntmachung des Hinweises auf die Patenterteilung: **11.12.2002**
(21) Anmeldenummer: 98938651.1
(22) Anmeldetag: 25.06.1998
(51) Int. Cl.: C07C 45/50, C07C 47/02, C07C 47/32, C07C 47/33, B01J 31/12, C07F 15/00

(54) **VERFAHREN ZUR HERSTELLUNG VON ALKANALEN MIT HILFE EINES RHODIUM-TRI-POLYETHYLENGLYKOLATS, UND DIESE VERBINDUNG SELBST**
METHOD FOR PRODUCING ALKANALS USING A RHODIUM-TRI-POLYETHYLENE GLYCOLATE
PROCEDE DE PRODUCTION D'ALCANALS AU MOYEN D'UN TRIPOLYETHYLENEGLYCOLATE DE RHODIUM, ET ALCANAL AINSI OBTENU

(30) Priorität: 07.07.1997 DE 19728944
(43) Veröffentlichungstag der Anmeldung: 10.05.2000
(73) Patentinhaber: Celanese Chemicals Europe GmbH, 60439 Frankfurt am Main (DE)
(72) Erfinder: BOGDANOVIC, Sandra, D-60322 Frankfurt am Main (DE); ROESKY, Herbert, D-37085 Göttingen (DE); RITTER, Uwe, D-37075 Göttingen (DE); BORRMANN, Thomas, D-37077 Göttingen (DE)
(86) Internationale Anmeldenummer: EP9803896
(87) Internationale Veröffentlichungsnummer: WO99002477

(56) Entgegenhaltungen:
- FR-A- 2 291 960
- US-A- 4 329 511
- CHEMICAL ABSTRACTS, vol. 125, no. 3, 15. Juli 1996 Columbus, Ohio, US; abstract no. 033125, YAN Y Y ET AL: "Aqueous-phase rhodium hydroformylation of dodecene-1 with surface-active water-soluble phosphine" XP000664491 & CHIN. CHEM. LETT. (CCLEE7);96; VOL.7 (4); PP.377-80, Dalian Univ. of Technology;Coll. of Chemical Engineering; Dalian; 116012; Peop. Rep. China (CN)

## Beschreibung

Die Erfindung liegt auf dem technischen Gebiet der Hydroformylierung von Olefinen.

Mit der vorliegenden Erfindung wurde die Aufgabe gelöst, insbesondere höhermolekulare lineare und verzweigte wie auch cyclische Alkanale durch Hydroformylierung von entsprechenden Olefinen mittels einem Rhodium-tri polyethylenglykolat als Katalysator hoher Aktivität in hoher Ausbeute und Reinheit herzustellen und sie aus dem Reaktionsansatz in einfacher Weise zu isolieren.

Die Hydroformylierung von Olefinen zu Aldehyden mit Hilfe eines Edelmetall-Komplexes ist bekannt. So ist in der U.S.-Patentschrift 4 329 511 die Umsetzung von Olefinen mit Wasserstoff und Kohlenmonoxid zu Aldehyden in inerten hochsiedenden Lösemitteln beschrieben, wobei als Katalysator ein Edelmetall der 8. Nebengruppe mit beispielsweise einem Triorganophosphin, wie Triphenylphosphin, komplex gebunden dient. Das hochsiedende Lösemittel muß ein Molekulargewicht von mindestens 700 bei Ethylen als Ausgangsolefin, von mindestens 1500 bei den höheren Olefinen besitzen. Diese hochsiedenden Lösemittel sollen den Katalysator lösen und bewirken, daß der Katalysator wiederverwendet werden kann, nachdem das gebildete Alkanal durch Destillation oder durch Austreiben mit einem inerten Gas aus dem Reaktionsansatz isoliert wurde. Diese Verfahrensweise hat jedoch, wie alle einphasig geführten Hydroformylierungsreaktionen von Olefinen unter Einsatz eines Metallkatalystors, den erheblichen Nachteil, daß die Abtrennung des Alkanals, insbesondere eines solchen mit einer C-Zahl von über 10, durch Destillation wegen seines hohen Siedepunktes nur mit erheblichem Aufwand, wenn überhaupt, möglich ist; denn neben Verlusten an dem Alkanal-Endprodukt treten nicht geringe Anteile an Schwerölen als Zersetzungsprodukte auf. Das in dieser U.S.-Patentschrift 4 329 511 beschriebene Hydroformylierungsverfahren ist deshalb nur zur Synthese von niederen Alkanalen, wie solchen von bis zu 7 C-Atomen, ökonomisch sinnvoll. Um höhermolekulare, hochsiedende Alkanale aus dem Reaktionsgemisch auszutreiben, müssen die Reaktionsgase (Kohlenmonoxid, Wasserstoff und Olefin) eine Temperatur von über 250°C besitzen.
In ähnlicher Weise wird die Hydroformylierung von Olefinen nach den Verfahren der europäischen Patentanmeidungs-Veröffentlichung Nr. 0 314 435 und der U.S.-Patentschrift 4 613 701 geführt, bei denen, in Konsequenz auf die hier diskutierten Nachteile, auch nur niedermolekulare Olefine in die Umsetzung eingesetzt werden.

Weiterhin beschreibt die deutsche Offenlegungsschrift Nr. 2 552 351 die Umsetzung von Olefinen mit Wasserstoff und Kohlenmonoxid in Gegenwart eines Rhodiumsalzes, wie Rhodiumchlorid, Rhodiumsulfat und Rhodiumnitrat, in Wasser oder einem Alkanol als Reaktionsmedium und Lösemittel unter Zusatz eines niederen Polyethylenglykols mit bis zu drei Ethoxyeinheiten, wobei dieses niedere Polyethylenglykol dazu dienen soll, eine Ausfällung des Rhodiumsalzes aus der Reaktionslösung zu verhindern. Auch diese Verfahrensweise wird einphasig geführt und hat die bereits erwähnten Nachteile.

Hingegen bieten zweiphasige Katalysesysteme unter Verwendung eines Lösemittels, das das gebildete Alkanal nicht löst, technische Vorteile. Die den Katalysator enthaltende Phase kann von dem gebildeten Alkanal ohne zusätzliche Verfahrensschritte abgetrennt werden. Zudem ist es nicht unbedingt erforderlich, den Katalysator nach der Reaktion zu isolieren, falls die polare Phase, die den Katalysator enthält, in einem kontinuierlichen Hydroformylierungsprozeß eingesetzt werden kann, bei welchem gebildetes Alkanal auch während des Prozeßverlaufes abgetrennt werden kann. Solch eine Verfahrensweise ist beispielsweise aus der deutschen Offenlegungsschrift 2 627 354 bekannt, bei welchem lineare Olefine unter Einwirkung eines sulfogruppenhaltigen Rhodium-triphenylphosphin-Komplexes in Wasser als Lösemittel hydroformyliert wird, Diese Verfahrensweise erlaubt jedoch nicht die Umsetzung von längerkettigen Olefinen, wie solchen von über 5 C-Atomen, da die Ausbeute der daraus erhältlichen Alkanale wegen der bei den höhermolekularen Olefinen nicht ausreichend Aktivität des Katalysators unbefriedigend ist. Außerdem muß das Phosphin, das der Komplexbildung mit dem Rhodium dient, in erheblichem Überschuß, wie bis zum 100-fachen Überschuß, bezogen auf das Rhodium, eingesetzt werden.

Mit der vorliegenden Erfindung wurde nunmehr eine zweiphasige Hydroformylierungsreaktion von Olefinen mit Hilfe eines neuen Katalysators gefunden, die die beschriebenen Nachteile des Standes der Technik umgeht und auch höhermolekulare lineare und verzweigte wie auch cyclische Olefine in hoher Ausbeute und Reinheit der Hydroformylierung zugänglich macht und es ermöglicht, daß die gebildeten Alkanale leicht, auch in einem kontinuierlichen Prozeß, aus dem Reaktionsansatz abgetrennt werden können.

Die vorliegende Erfindung betrifft somit die Herstellung von linearen und verzweigten aliphatischen Monoaldehyden (Alkanalen) von 6 bis 21 C-Atomen, bevorzugt von 7 bis 19 C-Atomen, und von cyclischen aliphatischen Monoaldehyden mit 6 bis 13 C-Atomen durch Hydroformylierung von linearen und verzweigten aliphatischen Monoolefinen (Alkenen) von 5 bis 20 C-Atomen, vorzugsweise von 6 bis 18 C-Atomen, bzw. von cyclischen Olefinen mit 5 bis 12 C-Atomen, d.h. durch Umsetzung solcher Olefine mit einem Kohlenmonoxid/Wasserstoff-Gasgemisch, mit Hilfe eines Rhodium-Katalysators, das dadurch gekennzeichnet ist, daß man die Umsetzung in heterogener Phase mittels einem Rhodium-tri-polyethylenglykolat eines Polyethylenglykols mit einem mittleren Molekulargewicht von 320 bis 650, bevorzugt von 350 bis 450, insbesondere bevorzugt von 400, bei einer Temperatur zwischen 50 und 150°C, bevorzugt zwischen 80 und 120°C, und bei einem Druck zwischen 60 und 200 bar, bevorzugt zwischen 75 und 120 bar, durchführt. Bevorzugt wird das Rhodium-tri-polyethylenglykolat in Form einer Lösung in Wasser, in Polyethylenglykol des angegebenen mittleren Molekulargewicht oder einem Gemisch dieses Polyethylenglykols mit Wasser als Lösemittel in die Reaktion eingesetzt.

Das als Katalysator dienende Rhodium-tri-polyethylenglykolat stellt eine neue Verbindung dar, in welcher das Polyethylenglykol an das Rhodium als Glykolat gebunden ist. Die vorliegende Erfindung betrifft somit auch Rhodium-tri-polyethylenglykolat-Verbindungen, wobei der Polyethylenglykol-Anteil ein mittleres Molekulargewicht von 320 bis 650, bevorzugt 350 bis 450, insbesondere bevorzugt ein mittleres Molekulargewicht von 400, besitzt. Die Erfindung betrifft weiterhin Lösungen eines erfindungsgemäßen Rhodium-tri-polyethylenglykolats in Wasser, in Polyethylenglykol oder in einem Polyethylenglykol/Wasser-Gemisch als Lösemittel, jeweils mit einem Polyethylenglykol mit einem mittleren Molekulargewicht von 320 bis 650, bevorzugt von 350 bis 450, insbesondere bevorzugt von 400.

Den neuen Rhodium-tri-polyethylenglykolat-Verbindungen kann die allgemeine Formel (1) 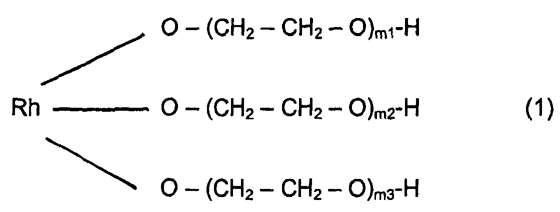 mit m₁, m₂ und m₃, jedes zueinander gleich oder voneinander verschieden, einer Zahl zwischen 6 und 15, bevorzugt zwischen 7 und 11, zugeordnet werden, wobei der dritte Teil der Summe von (m₁ + m₂ + m₃) zwischen etwa 6,8 und etwa 14,4, bevorzugt zwischen etwa 7,55 und etwa 9,8, liegt.
Die erfindungsgemäße Rhodium-tri-polyethylenglykolat-Verbindung läßt sich vorteilhaft in der Weise herstellen, indem man Rhodium(III)-chlorid-Trihydrat mit dem Polyethylenglykol in stöchiometrischer Menge (d.h. in der 3-fach äquivalenter Menge) bei einer Temperatur zwischen 40 und 80°C, bevorzugt zwischen 50 und 65°C, vorteilhaft unter einem Stickstoffstrom und gegebenenfalls unter leicht reduziertem Druck, erhitzt, wobei das gebildete Chlorwasserstoffgas entfernt wird. Es stellt eine einheitliche Verbindung dar. Es ist mit Wasser und einem Polyethylenglykol mit mittlerem Molekulargewicht von 320 bis 650 gut mischbar. Seine Synthese ist unter gleichen Bedingungen auch mit einem Überschuß von Polyethylenglykol möglich, wobei sogleich die Lösung des Rhodium-tripolyethylenglykolats in Polyethylenglykol entsteht, die vorteilhaft, gegebenenfalls vermischt mit Wasser, in die Hydroformylierungsreaktion eingesetzt werden kann.

Olefine, die in den erfindungsgemäßen Hydroformylierungsprozeß eingesetzt werden, sind beispielsweise 1-Hexen, 1-Octen, 1-Decen, 1-Dodecen, 1-Tetradecen, 2-Hexen, 2-Hepten, 2-Octen, 2,4,4-Trimethyl-1-penten, 2,4,4-Trimethyl-2-penten, Cyclohexen, Cycloocten und 4-Methyl-1-cyclohexen.

Die erfindungsgemäße Hydroformylierung erfolgt in der Regel in der Weise, daß man das Rhodium-tri-polyethylenglykolat, gelöst in einem gegebenenfalls mit Wasser vermischten Polyethylenglykol mit dem angegebenen durchschnittlichen Molekulargewicht oder in Wasser alleine, in einem Autoklaven vorlegt, ein Kohlenmonoxid/Wasserstoff-Gasgemisch innerhalb des angegebenen bar-Bereiches in den Gasraum des Autoklaven aufdrückt, wobei der Autoklav vor,
während oder nach der Einspeisung des Kohlenmonoxid/Vasserstoff-Gasgemisches auf die gewünschte Reaktionstemperatur erhitzt wird, und anschließend unter weiterem Rühren der Lösung das Olefin unter Einhaltung des Reaktionsdruckes in den Reaktionsansatz einführt. Die so erhaltene Reaktionsmischung wird sodann einige Stunden lang bei der gewünschten Reaktionstemperatur und dem Reaktionsdruck unter weiterem Rühren umgesetzt. Nach Abkühlen des Autoklaven und Entspannen wird aus dem gebildeten Ansatz die Aldehydphase von der Phase der Polyethylenglykollösung bzw. der wäßrigen Phase abgetrennt. Bei geeignet konstruiertem Autoklaven kann die Hydroformylierung jedoch auch kontinuierlich erfolgen, d.h. durch kontinuierliche Zugabe der Reaktanten zu der Rhodium-tripolyethylenglykolat-Lösung und unter kontinuierlicher Abtrennung der gebildeten Aldehydphase. Bei der diskontinuierlichen Verfahrensweise kann die abgetrennte Katalysatorphase wieder in den Autoklaven zur Durchführung eines weiteren Reaktionsansatzes zurückgeführt werden.

Die nach Beendigung der Umsetzung abtrennbare Aldehydphase besteht aus praktisch reinem Aldehyd (ein Übergang von Rhodium in die Aldehydphase ist praktisch nicht nachweisbar), der für die Weiterverwendung nicht weiter, beispielsweise durch Destillation, gereinigt werden muß. Diesbezüglich ist das erfindungsgemäße Verfahren besonders von Vorteil zur Synthese von höhersiedenden Alkanalen, wie solchen mit mehr als 10 C-Atomen.

1-Olefine, d.h. Olefine mit endständiger Doppelbindung, liefern bei der Hydroformulierung ein Gemisch von isomeren Alkanalen, bei denen die Aldehydgruppe in 1- und in 2-Stellung des aliphatischen Restes steht. In der Regel läßt sich durch Zusatz von bestimmten sekundären und tertiären Aminen sowie cyclischen Aminen bei der Hydroformylierungsreaktion eine Selektivität zugunsten des 1-Alkanals erreichen. Solche Verbindungen sind beispielsweise Piperidin, Pyridin, 3-Methyl-pyridin, Dialkylamine mit Alkylresten von jeweils 1 bis 4 C-Atomen, wie Dimethylamin und insbesondere Diethylamin und Dipropylamin, sowie Trialkylamine mit Alkylresten von jeweils 1 bis 4 C-Atomen, wie Triethylamin. Diese Amine werden in der Regel in einer Menge von bis zu 0,5 Gew.-%, bezogen auf das Olefin, eingesetzt.

Das zur Umsetzung eingesetzte Kohlenmonoxid/\Wasserstoff-Gasgemisch liegt in der Regel in äquimolaren Mengen der Wasserstoff- und Kohlenmonoxid-Anteile vor, wobei einer dieser Reaktanten ohne Nachteile auch bis zu einem 50 %igen Überschuß, Wasserstoff gegebenenfalls sogar bis zu einem 100 %igen Überschuß, in dem Gasgemisch vorliegen kann.

In der Regel liegt das in den Autoklaven eingeführte Kohlenmonoxid/Wasserstoff-Gasgemisch je nach Wahl des entsprechenden Gasgemischdruckes in Bezug auf das eingesetzte Olefin im bis zu 2-molaren Überschuß in dem Reaktionsraum vor. Zwar können die Reaktanten zu Anfang auch im molaren Verhältnis zum Olefin in dem Reaktionsansatz vorliegen, jedoch ist die Anwesenheit der Reaktanten Wasserstoff und Kohlenmonoxid im Überschuß von Vorteil, zumal sie sich wieder leicht aus dem Autoklaven nach Beendigung der Reaktion entfernen lassen.

Das Gewichtsverhältnis zwischen der Wasser-, Polyethylenglykol- bzw. Polyethylenglykol/ Wasser-Phase, die das Rhodium-tri-polyethylenglykolat gelöst enthält, und dem Olefin kann zu Anfang der Reaktion zwischen 10: 1 und 1 : 3 sein; besonders vorteilhaft hat sich ein Gewichtsverhältnis zwischen 2 : 1 und 1 : 2 erwiesen.
Das molare Verhältnis des Rhodium-tri-polyethylenglykolat-Katalysators in der gegebenenfalls wasserhaltigen Polyethylenglykolphase zu dem Olefin läßt sich durch den Anteil des Polyethylenglykols bzw. Polyethylenglykol/Wasser-Gemisches als Lösemittel steuern. Bezogen auf das Rhodium selbst kann das Molverhältnis zum Olefin bei 1 : 900 bis 1 : 20000 liegen. Bevorzugt hierbei ist ein molares Verhältnis zwsichen Rhodium zu Olefin am Beginn der Reaktion im Bereich von 1 : 1800 bis 1 : 12000, bevorzugt von 1 : 4000 bis 1 : 10000, bei verzweigten Olefinen im allgemeinen vorteilhaft auch ein Verhältnis von 1 : 1800 bis 1 : 5000.

Die Reaktionszeit der Hydroformylierung des Olefins im erfindungsgemäßen Verfahren kann mehrere Stunden betragen und kann, abhängig von Reaktionstemperatur, Reaktionsdruck und dem spezifischen Olefin, zwischen 2 und 15 Stunden liegen.

Der Einsatz des erfindungsgemäßen Rhodium-tri-polyethylenglykolat-Katalysators hat den Vorteil, daß dessen Aktivität auch bei höherkettigen Olefinen, wie solchen mit mehr als 9 C-Atomen, nicht abnimmt. Zudem können auch verzweigte Olefine und Olefingemische, die erfahrungsgemäß schwerer zu hydroformylieren sind als lineare Olefine, erfindungsgemäß mit besonderem Vorteil hydroformyliert werden.

Die nachstehenden Beispiele dienen zur Erläuterung der Erfindung, Die Prozentangaben beziehen sich auf Gewichtsprozente und die Teile sind Gewichtsteile, sofern nicht anders angegeben. Teile stehen zu Volumenteilen im Verhältnis wie Kilogramm zu Liter.

### Beispiel A

Zur Synthese des reinen Rhodium-tri-polyethylenglykolats wird Rhodium(III)-chlorid-Trihydrat mit Polyethylenglykol von einem durchschnittlichen Molekulargewicht von 400 in stöchiometrischen Mengen umsetzt:
10 Teile Rhodium(lll)-chlorid-Trihydrat werden mit 16 Vol.-Teilen Polyethylenglykol 400 unter Überleiten von Stickstoff auf 60°C erwärmt, und der Ansatz wird zwei Tage unter einem Stickstoffstrom bei dieser Temperatur gehalten.
Die erhaltene Verbindung ist ein dunkelrotes Öl von zähflüssiger bis fester Konsistenz. Es ist mit Wasser unbegrenzt mischbar, ebenso mit Polyethylenglykol, und löst sich in Acetonitril. Hält man das Öl bei leichtem Erwärmen auf 50°C unter reduziertem Druck von 0,01 bar während etwa acht Stunden, so läßt sich restliches Chlorwasserstoffgas, das sich bei der Reaktion gebildet hatte, praktisch vollständig entfernen. Das Rhodium-tri-polyethylenglykolat ist auf diese Weise praktisch chloridfrei.

### Analyse:

Rhodiumgehalt: 19,9 %, Chloridgehalt: 200 ppm;
IR-Spektrum (Film, KBr): 3449, 2098, 1956, 1740, 1649 cm⁻¹;
Absorptionsmaximum im UV/VIS-Bereich: 445 nm;
¹H-NMR (in d₇-Acetonitril; Referenz Tetramethylsilan);
   δ = 3,4 bis 3,6 ppm (Multipletts).

### Beispiel B

Zur Synthese einer Lösung von Rhodium-tri-polyethylenglykolat eines Polyethylenglykols 400 in Polyethylenglykol 400 als Lösemittel löst man 5 Teile Rhodium(III)-chlorid-Trihydrat in 150 Vol.-Teile Polyethylenglykol von einem mittleren Molekulargewicht von 400, rührt die Lösung während drei Stunden bei 40°C bei Überleiten eines leichten Stickstoffstromes zur Entfernung des gebildeten Chlorwasserstoffgases und reduziert sodann den Druck im Reaktionsgefäß auf 0,01 bar zur Entfernung restlicher Spuren von Chlorwasserstoffgas.

Die anschließende Analyse zeigt vollständige Umsetzung der Reaktion und daß die Lösung keine Chloridionen mehr enthält. Der Rhodiumgehalt der Lösung beträgt 13 Teile Rhodium pro Liter Polyethylenglykol 400.

### Beispiel 1

5 Vol.-Teile der nach Beispiel B hergestellten Lösung des Rhodium-Katalysators werden mit 195 Vol.-Teilen Polyethylenglykol 400 mit einem Wassergehalt von 1 % vermischt und in einen Autoklaven gegeben, dessen Gasraum sodann mit einem äquimolaren Gemisch aus Wasserstoff und Kohlenmonoxid unter einem Gesamtdruck von 80 bar gefüllt wird. Man erwärmt die Lösung unter Rühren auf 100°C, hält die Temperatur bei weiterem Rühren und unter Einhaltung des Druckes von 80 bar noch etwa drei Stunden und gibt sodann unter weiterer Einhaltung der bisherigen Reaktionsbedingungen 200 Vol.-Teile 1-Hexen hinzu, rührt den Reaktionsansatz noch weitere zwei Stunden und überführt nach Abkühlen und Entspannen des Autoklaven das erhaltene Reaktionsgemisch in einen Phasenseparator. Man trennt die obere Phase, die aus dem synthetisiertem Aldehyd besteht, von der unteren Phase, der den Rhodium-Katalysator enthaltenden Polyethylenglykollösung, die für einen erneuten Ansatz in den Autoklaven zurückgeführt werden kann.
Eine gaschromatographische Analyse der abgetrennten Produktphase ergibt einen Gehalt von 98 % Heptanal; die restlichen 2 % bestehen überwiegend aus nicht umgesetzten 1-Hexen und aus isomerisiertem Hexen (2-Hexen). Das Verhältnis von 1-Heptanal zu 2-Heptanal, d.h. linearem zu verzweigtem Heptanal, beträgt 0,75. Die Ausbeute an Heptanal beträgt 98 % d.Th..

### Beispiel 2

Man verfährt gemäß der Verfahrensweise des Beispieles 1, setzt jedoch anstelle von 1-Hexen die gleiche Menge an 1-Octen und anstelle von 5 Vol.-Teilen der Lösung des Rhodium-Katalysators 10 Vol.-Teile davon in den Reaktionsansatz ein. Nach Beendigung der Reaktion wird die Aldehydphase abgetrennt. Sie enthält zu 99 % ein Gemisch aus 1-Nonanal und 2-Nonanal in einer Ausbeute von 99 % d.Th. bei einem molaren Verhältnis von 1- zu 2-Nonanal von 0,5.

### Beispiel 3

Man verfährt zur Hydroformylierung von 1-Octen in der im Beispiel 2 angegebenen Verfahrensweise, setzt jedoch zusätzlich mit dem 1-Octen 0,002 Teile Pyridin dem Reaktionsansatz zu.
Nach Abtrennung der Nonanal-Phase erhält man ein 1-Nonanal/2-Nonanal-Gemisch im Verhältnis von 1,7 in einer Ausbeute von 89 % d.Th. bei einer Reinheit von 89 % (die restlichen Anteile bestehen im wesentlichen aus nicht umgesetztem 1-Octen und 2-Octen). Der Zusatz von Pyridin hat somit die Selektivität zum linearen Nonanal begünstigt.

### Beispiel 4

Man verfährt zur Hydroformylierung von 1-Octen in der im Beispiel 1 angegebenen Verfahrensweise, setzt jedoch zusätzlich mit dem 1-Octen 0,005 Teile Pyridin dem Reaktionsansatz zu.
Nach Abtrennung der Nonanal-Phase erhält man ein 1-Nonanal/2-Nonanal-Gemisch im Verhältnis von 1,9 in einer Ausbeute von 96 % d.Th. bei einer Reinheit von 96 % (die restlichen 4 % bestehen im wesentlichen aus nicht umgesetztem 1-Octen und 2-Octen). Der Zusatz von Pyridin hat somit die Selektivität zum linearen Nonanal begünstigt.

### Beispiel 5

a) Zur Synthese einer Lösung von Rhodium-tri-polyethylenglykolat eines Polyethylenglykols 600 in Polyethylenglykol 600 als Lösemittel löst man 10 Teile Rhodium(III)-chlorid-Trihydrat in 150 Vol.-Teilen Polyethylenglykol von einem mittleren Molekulargewicht von 600, rührt die Lösung während drei Stunden bei 40°C bei Überleiten eines leichten Stickstoffstromes zur Entfernung des gebildeten Chlorwasserstoffgases und reduziert sodann den Druck des Reaktionsgefäßes auf 0,01 bar zur Entfernung restlicher Spuren von Chlorwasserstoffgas.
b) 10 Vol.-Teile der so hergestellten Lösung des Rhodium-Katalysators werden mit 195 Vol.-Teilen Polyethylenglykol 600 mit einem Wassergehalt von 2 % vermischt und in einen Autoklaven gegeben. Dessen Gasraum wird mit einem äquimolaren Gemisch aus Wasserstoff und Kohlenmonoxid unter einem Gesamtdruck von 80 bar gefüllt. Man erwärmt den Ansatz unter Rühren auf 100°C, hält die Temperatur bei weiterem Rühren und unter Einhaltung des Druckes von 80 bar noch etwa drei Stunden, gibt sodann unter Einhaltung der bisherigen Reaktionsbedingungen 200 Vol.-Teile 1-Octen hinzu und rührt den Reaktionsansatz noch weitere zwei Stunden. Nach Abkühlen und Entspannen des Autoklaven trennt man die Aldehydphase ab, die das Nonanal zu 79 % enthält (die restlichen 21 % bestehen überwiegend aus nicht umgesetzten 1-Octen und isomerisiertem Octen). Das Verhältnis von 1-Nonanal zu 2-Nonanal, d.h. linearem zu verzweigtem Nonanal, beträgt 1,2. Die Ausbeute an Nonanal beträgt 79 % d.Th..

### Beispiel 6

10 Vol.-Teile der nach Beispiel B hergestellten Lösung des Rhodium-Katalysators werden mit 195 Vol.-Teile Polyethylenglykol 400 mit einem Wassergehalt von 2 % vermischt und in einen Autoklaven gegeben, dessen Gasraum sodann mit einem äquimolaren Gemisch aus Wasserstoff und Kohlenmonoxid unter einem Gesamtdruck von 80 bar gefüllt wird. Man erwärmt die Lösung unter Rühren auf 100°C, hält die Temperatur bei weiterem Rühren und unter Einhaltung des Druckes von 80 bar noch etwa drei Stunden, gibt sodann unter Einhaltung einer Temperatur von etwa 100°C und eines Druckes von etwa 80 bar 200 Vol.-Teilen 1-Dodecen hinzu und rührt den Reaktionsansatz noch weitere zwei Stunden weiter. Nach Abkühlen und Entspannen des Autoklaven wird die Aldehydphase abgetrennt.
Die gaschromatographische Analyse der abgetrennten Produktphase ergibt einen Gehalt an Tridecanal von 99 %; die restlichen Teile bestehen überwiegend aus nicht umgesetztem 1-Dodecen und durch Isomerisierung entstandenem 2-Dodecen. Das Verhältnis von 1-Tridecanal zu 2-Tridecanal, d.h. linearem zu verzweigtem Tridecanal, beträgt 1,1. Die Ausbeute an Tridecanal beträgt 99,0 % d.Th.

### Beispiel 7

Man verfährt zur Hydroformylierung von 1-Dodecen in der im Beispiel 5b) angegebenen Verfahrensweise, wobei man 1-Dodecen in gleicher Menge anstelle des 1-Octens einsetzt. Nach Abtrennung der Tridecanal-Phase erhält man ein 1-Tridecanal/2-Tridecanal-Gemisch im Verhältnis von 0,2 in einer Ausbeute von 88,0 % d.Th. bei einer Reinheit von 88 %.

### Beispiel 8

20 Vol.-Teile der nach Beispiel B hergestellten Lösung des Rhodium-Katalysators werden mit 800 Vol.-Teilen Polyethylenglykol 400 mit einem Wassergehalt von 2 % vermischt und in einen Autoklaven gegeben, dessen Gasraum sodann mit einem äquimolaren Gemisch aus Wasserstoff und Kohlenmonoxid unter einem Gesamtdruck von 80 bar gefüllt wird. Man erwärmt die Lösung unter Rühren auf 100°C, hält die Temperatur bei weiterem Rühren und unter Einhaltung des Druckes von 80 bar noch etwa drei Stunden und gibt sodann unter Einhaltung dieser Reaktionsbedingungen 800 Vol.-Teile 2-Hexen hinzu, rührt den Reaktionsansatz noch weitere zwei Stunden bei 100°C und 80 bar und überführt nach Abkühlen und Entspannen des Autoklaven das erhaltene Reaktionsgemisch in einen Phasenseparator, in dem man die Aldehyd-Phase mit einem Heptanal-Anteil von 96 % abtrennt.
Infolge einer teilweisen Isomerisierung von 2-Hexen zu 1-Hexen ergibt sich ein Gemisch aus 1-Heptanal und 2-Heptanal im Mischungsverhältnis von 0,83. Die Ausbeute an dem Heptanal-Gemisch beträgt 96 % d.Th.

### Beispiel 9

Man verfährt gemäß der Verfahrensweise des Beispieles 8, setzt jedoch anstelle des 2-Hexens die gleiche Menge an einem Gemisch aus 1-Hexen und 2-Hexen im Mischungsverhältnis von 1 : 1 sowie anstelle von 5 Vol.-Teilen der nach Beispiel B hergestellten Lösung des Rhodium-Katalysators 10 Vol.-Teile hiervon in den Reaktionsansatz ein. Man erhält eine Mischung aus 1-Heptanal und 2-Heptanal im Verhältnis von 0,9 bei einer Ausbeute von 94 % d.Th..

### Beispiel 1 0

4 Vol.-Teile der nach Beispiel B hergestellten Lösung des Rhodium-Katalysators werden mit 300 Vol.-Teilen Wasser vermischt und in einen Autoklaven gegeben, dessen Gasraum sodann mit einem äquimolaren Gemisch aus Wasserstoff und Kohlenmonoxid unter einem Gesamtdruck von 100 bar gefüllt wird. Man erwärmt den Ansatz unter Rühren auf 100°C, hält die Temperatur bei weiterem Rühren und unter Einhaltung des Druckes von 100 bar noch etwa drei Stunden und gibt sodann unter dieser Bedingung 320 Vol.-Teile 2,4,4-Trimethyl-1-penten hinzu. Man hält unter weiterem Rühren das Reaktionsgemisch noch weitere fünf Stunden bei 100°C und überführt nach Abkühlen und Entspannen des Autoklaven das erhaltene Reaktionsgemisch in einen Phasenseparator. Man trennt die Aldehyd-Phase von der wäßrigen, den Rhodium-Katalysator enthaltenden Phase ab. die für einen erneuten Ansatz in den Autoklaven zurückgeführt werden kann. Die gaschromatographische Analyse der abgetrennten Produktphase ergibt folgende Zusammensetzung:

| | | | |
|---|---|---|---|
| 3,5,5-Trimethyl-1-hexanal | 92 %; | 2-t-Butyl-3-methyl-1-butanal | 0,5 %; |
| 2,4,4-Trimethyl-1-penten | 3,4 %; | 2,4,4-Trimethyl-2-penten | 1,2 %; |
| 2,4,4-Trimethyl-pentan | 1,2 %; | 3,5,5-Trimethyl-1-hexanol | 0,4 %; |
| nicht flüchtige Verbindungen | 1,2 %. | | |

### Beispiel 11

Man verfährt gemäß der Verfahrensweise des Beispieles 10, setzt jedoch anstelle des dort verwendeten Olefins dessen isomere Verbindung 2,4,4-Trimethyl-2-penten in gleicher Menge ein.
Die gaschromatographische Analyse der abgetrennten Aldehydphase ergibt folgende Zusammensetzung:

| | | | |
|---|---|---|---|
| 3,5,5-Trimethyl-1-hexanal | 91 %; | 2-t-Butyl-3-methyl-1-butanal | 2,5 %; |
| 2,4,4-Trimethyl-1-penten | 1,5 %; | 2,4,4-Trimethyl-2-penten | 3,2 %; |
| 2,4,4-Trimethyl-pentan | 0,2 %; | 3,5,5-Trimethyl-1-hexanol | 0,4 %; |
| nicht flüchtige Verbindungen | 1,2 %. | | |

### Beispiel 12

Man verfährt gemäß der Verfahrensweise des Beispieles 10, setzt jedoch anstelle des 2,4,4-Trimethyl-1-pentens ein als Diisobutylen bezeichnetes technisches Gemisch aus 76 % 2,4,4-Trimethyl-1-penten und 24 % 2,4,4-Trimethyl-2-penten in gleicher Menge ein.
Die abgetrennte Aldehydphase zeigt folgende Zusammensetzung:

| | | | |
|---|---|---|---|
| 3,5,5-Trimethyl-1 -hexanal | 93 %; | 2-t-Butyl-3-methyl-1-butanal | 1,5 %; |
| 2,4,4-Trimethyl-1-penten | 1,5 %; | 2,4,4-Trimethyl-2-penten | 2,2 %; |
| 2,4,4-Trimethyl-pentan | 0,2 %; | 3,5,5-Trimethyl-1-hexanol | 0,4%; |
| nicht flüchtige Verbindungen | 1,2 %. | | |

## Patentansprüche

1. Verfahren zur Herstellung von linearen und verzweigten aliphatischen Monoaldehyden von 6 bis 21 C-Atomen und von cyclischen aliphatischen Monoaldehyden mit 6 bis 13 C-Atomen durch Umsetzung von linearen und verzweigten aliphatischen Monoolefinen von 5 bis 20 C-Atomen bzw. von cyclischen Olefinen mit 5 bis 12 C-Atomen mit einem Kohlenmonoxid/-Wasserstoff-Gasgemisch mit Hilfe eines Rhodiumkatalysators, **dadurch gekennzeichnet, daß** man die Umsetzung in heterogener Phase mittels einem Rhodium-tri-polyethylenglykolat eines Polyethylenglykols mit einem mittleren Molekulargewicht von 320 bis 650 bei einer Temperatur zwischen 50 und 150°C und einem Druck zwischen 60 und 200 bar durchführt.

2. Verfahren nach Anspruch 1, **dadurch gekennzeichnet, daß** man die Umsetzung in Wasser, in einem Polyethylenglykol oder Polyethylenglykol/Wasser-Gemisch, jeweils eines Polyethylenglykols mit einem mittleren Molekulargewicht von 320 bis 650, als Lösemittel durchführt.

3. Verfahren nach Anspruch 1 oder 2, **dadurch gekennzeichnet, daß** man ein lineares oder verzweigtes Monoolefin von 6 bis 18 C-Atomen in die Reaktion einsetzt.

4. Verfahren nach Anspruch 1 oder 2, **dadurch gekennzeichnet, daß** man ein lineares oder verzweigtes Monoolefin von 10 bis 20 C-Atomen in die Reaktion einsetzt.

5. Verfahren nach mindestens einem der Ansprüche 1 bis 4, **dadurch gekennzeichnet, daß** das Polyethylenglykol bzw. -glykolat ein mittleres Molekulargewicht von 350 bis 450 besitzt.

6. Verfahren nach mindestens einem der Ansprüche 1 bis 5, **dadurch gekennzeichnet, daß** man die Umsetzung bei einer Temperatur zwischen 80 und 120°C durchführt.

7. Verfahren nach mindestens einem der Ansprüche 1 bis 6, **dadurch gekennzeichnet, daß** man die Umsetzung bei einem Druck zwischen 75 und 120 bar durchführt.

8. Verfahren nach mindestens einem der Ansprüche 1 bis 7, **dadurch gekennzeichnet, daß** man die Umsetzung in Gegenwart von bis zu 0,5 Gew.-%, bezogen auf das Olefin, eines sekundären oder tertiären Amins oder eines cyclischen Amins durchführt.

9. Verfahren nach Anspruch 8, **dadurch gekennzeichnet, daß** das Amin ein Dialkylamin mit Alkylresten von jeweils 1 bis 4 C-Atomen oder ein Trialkylamin mit Alkylrest von jeweils 1 bis 4 C-Atomen ist.

10. Verfahren nach Anspruch 8, **dadurch gekennzeichnet, daß** das Amin Piperidin, Pyridin oder 3-Methyl-pyridin ist.

11. Ein Rhodium-tri-polyethylenglykolat eines Polyethylenglykols mit einem mittleren Molekulargewicht von 320 bis 650.

12. Ein Rhodium-tri-polyethylenglykolat nach Anspruch 11 mit einem Polyethylenglykol mit einem mittleren Molekulargewicht von 350 bis 450.

13. Verfahren zur Herstellung eines Rhodium-tri-polyethylenglykolats nach Anspruch 11, **dadurch gekennzeichnet, daß** man Rhodium(III)-chlorid-Trihydrat mit einem Polyethylenglykol mit einem mittlerem Molekulargewicht von 320 bis 650 in stöchiometrischer Menge bei einer Temperatur zwischen 40 und 80°C erhitzt.

14. Lösung eines Rhodium-tri-polyethylenglykolats von Anspruch 11 in Wasser, in Polyethylenglykol mit einem mittleren Molekulargewicht von 320 bis 650 oder in einer Mischung davon.

15. Verwendung eines Rhodium-tri-polyethylenglykolats von Anspruch 11 oder 12 oder eines nach Anspruch 13 hergestellten Rhodium-tri-polyethylenglykolats als Katalysator zur Synthese von linearen und verzweigten Alkanalen und Cycloalkanalen durch Hydroformylierung von linearen und verzweigten Alkenen und Cycloalkenen.

## Claims

1. A process for preparing linear and branched aliphatic monoaldehydes having from 6 to 21 carbon atoms and cyclic aliphatic monoaldehydes having from 6 to 13 carbon atoms by reacting linear and branched aliphatic monoolefins having from 5 to 20 carbon atoms or cyclic olefins having from 5 to 12 carbon atoms with a carbon monoxide/hydrogen gas mixture using a rhodium catalyst, wherein the reaction is carried out in a heterogeneous phase by means of a rhodium (tripolyethylene glycolate) of a polyethylene glycol having a mean molecular weight of from 320 to 650 at a temperature of from 50 to 150°C and a pressure of from 60 to 200 bar.

2. The process as claimed in claim 1, wherein the reaction is carried out in water, in a polyethylene glycol or a polyethylene glycol/water mixture as solvent, in each case using a polyethylene glycol having a mean molecular weight of from 320 to 650.

3. The process as claimed in claim 1 or 2, wherein a linear or branched monoolefin having from 6 to 18 carbon atoms is used in the reaction.

4. The process as claimed in claim 1 or 2, wherein a linear or branched monoolefin having from 10 to 20 carbon atoms is used in the reaction.

5. The process as claimed in at least one of claims 1 to 4, wherein the polyethylene glycol or glycolate has a mean molecular weight of from 350 to 450.

6. The process as claimed in at least one of claims 1 to 5, wherein the reaction is carried out at a temperature of from 80 to 120°C.

7. The process as claimed in at least one of claims 1 to 6, wherein the reaction is carried out at a pressure of from 75 to 120 bar.

8. The process as claimed in at least one of claims 1 to 7, wherein the reaction is carried out in the presence of up to 0.5% by weight, based on the olefin, of a secondary or tertiary amine or a cyclic amine.

9. The process as claimed in claim 8, wherein the amine is a dialkylamine having alkyl radicals of from 1 to 4 carbon atoms each or a trialkylamine having alkyl radicals of from 1 to 4 carbon atoms each.

10. The process as claimed in claim 8, wherein the amine is piperidine, pyridine or 3-methylpyridine.

11. A rhodium tri(polyethylene glycolate) of a polyethylene glycol having a mean molecular weight of from 320 to 650.

12. A rhodium tri(polyethylene glycolate) as claimed in claim 11 derived from a polyethylene glycol having a mean molecular weight of from 350 to 450.

13. A process for preparing a rhodium tri(polyethylene glycolate) as claimed in claim 11, which comprises heating rhodium(III) chloride trihydrate at a temperature of from 40 to 80°C with a stoichiometric amount of a polyethylene glycol having a mean molecular weight of from 320 to 650.

14. A solution of a rhodium tri(polyethylene glycolate) as claimed in claim 11 in water, in polyethylene glycol having a mean molecular weight of from 320 to 650 or in a mixture thereof.

15. The use of a rhodium tri(polyethylene glycolate) as claimed in claim 11 or 12 or a rhodium tri(polyethylene glycolate) prepared as claimed in claim 13 as catalyst for the synthesis of linear and branched alkanals and cycloalkanals by hydroformylation of linear and branched alkenes and cycloalkenes.

## Revendications

1. Procédé pour la préparation de monoaldéhydes aliphatiques linéaires et ramifiés en C₆-C₂₁ et de monoaldéhydes aliphatiques cycliques en C₆-C₁₃ par réaction de mono-oléfines aliphatiques linéaires et ramifiées en C₅-C₂₀ et respectivement d'oléfines cycliques en C₅-C₁₂ avec un mélange gazeux monoxyde de carbone/hydrogène avec l'aide d'un catalyseur au rhodium, **caractérisé en ce que** la réaction est réalisée en phase hétérogène sur un tri-polyéthylèneglycolate de rhodium d'un polyéthylèneglycol de poids moléculaire moyen 320 à 650 à une température de 50 à 150°C et une pression de 60 à 200 bars.

2. Procédé selon la revendication 1, **caractérisé en ce que** la réaction est réalisée dans l'eau, un polyéthylèneglycol ou un mélange polyéthylèneglycol/eau, dans les deux cas avec un polyéthylèneglycol de poids moléculaire moyen 320 à 650.

3. Procédé selon la revendication 1 ou 2, **caractérisé en ce que** l'on met en oeuvre dans la réaction une mono-oléfine linéaire ou ramifiée en C₆-C₁₈.

4. Procédé selon la revendication 1 ou 2, **caractérisé en ce que** l'on met en oeuvre dans la réaction une mono-oléfine linéaire ou ramifiée en C₁₀-C₂₀.

5. Procédé selon au moins une des revendications 1 à 4, **caractérisé en ce que** le polyéthylèneglycol ou le polyéthylèneglycol à l'état de glycolate, a un poids moléculaire moyen de 350 à 450.

6. Procédé selon au moins une des revendications 1 à 5, **caractérisé en ce que** la réaction est réalisée à une température comprise entre 80 et 120°C.

7. Procédé selon au moins une des revendications 1 à 6, **caractérisé en ce que** la réaction est réalisée sous une pression comprise entre 75 et 120 bars.

8. Procédé selon au moins une des revendications 1 à 7, **caractérisé en ce que** la réaction est réalisée en présence d'une proportion allant jusqu'à 0,5 % en poids, par rapport à l'oléfine, d'une amine secondaire ou tertiaire ou d'une amine cyclique.

9. Procédé selon la revendication 8, **caractérisé en ce que** l'amine est une dialkylamine à groupes alkyle chacun en C₁-C₄ ou une trialkylamine à groupes alkyle chacun en C₁-C₄.

10. Procédé selon revendication 8 **caractérisé en ce que** l'amine est la pipéridine, la pyridine ou la 3-méthylpyridine.

11. Un tri-polyéthylèneglycolate de rhodium d'un polyéthylèneglycol de poids moléculaire moyen de 320 à 650.

12. Un tri-polyéthylèneglycolate de rhodium selon la revendication 11, ayant un polyéthylèneglycol au poids moléculaire moyen de 350 à 450.

13. Procédé pour la préparation d'un tri-polyéthylèneglycolate de rhodium selon revendication 11, **caractérisé en ce que** l'on chauffe le chlorure de rhodium (III) trihydraté avec un polyéthylèneglycol de poids moléculaire moyen 320 à 650 en quantité stoechiométrique à une température comprise entre 40 et 80°C.

14. Solution d'un tri-polyéthylèneglycolate de rhodium selon la revendication 11, dans l'eau, dans un polyéthylèneglycol de poids moléculaire moyen de 320 à 650, ou dans un mélange des deux.

15. Utilisation d'un tri-polyéthylèneglycolate de rhodium selon la revendication 11 ou 12, ou d'un tri-polyéthylèneglycolate de rhodium préparé selon la revendication 13 en tant que catalyseur pour la synthèse d'alcanals linéaires et ramifiés et de cycloalcanals par hydroformylation d'alcènes linéaires et ramifiés et de cycloalcènes.
